# EUROPEAN PATENT APPLICATION

(11) **EP 4 644 570 A1**
(43) Date of publication of application: **05.11.2025**
(21) Application number: 22969843.6
(22) Date of filing: 30.12.2022
(51) Int. Cl.: C12Q 1/6869, C12N 9/12, G01N 27/447

(54) **HELICASE MUTANT, AND PREPARATION METHOD THEREFOR AND USE THEREOF IN HIGH-THROUGHPUT SEQUENCING**

(71) Applicant: BGI Shenzhen, Shenzhen, Guangdong 518083 (CN); BGI Shenzhen Co., Ltd, Shenzhen, Guangdong 518083 (CN)
(72) Inventor: KONG, Chaodi, Shenzhen, Guangdong 518083 (CN); WANG, Lele, Shenzhen, Guangdong 518083 (CN); LIU, Zhenjun, Shenzhen, Guangdong 518083 (CN); WANG, Zi, Shenzhen, Guangdong 518083 (CN); WANG, Ou, Shenzhen, Guangdong 518083 (CN); SHI, Xiao, Shenzhen, Guangdong 518083 (CN); JI, Zhouxiang, Shenzhen, Guangdong 518083 (CN); GUO, Fei, Shenzhen, Guangdong 518083 (CN); ZENG, Tao, Shenzhen, Guangdong 518083 (CN); LI, Yuxiang, Shenzhen, Guangdong 518083 (CN); DONG, Yuliang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Dehns
(86) International application number: PCT/CN2022/144205
(87) International publication number: WO 2024/138701

(57) **Abstract**

Provided are a helicase mutant, a preparation method therefor, and use thereof in high-throughput sequencing. The helicase mutant has at least one cysteine on a surface of a three-dimensional structure of an amino acid sequence as set forth in SEQ ID NO: 1, the at least one cysteine being substituted with alanine. The cysteine is C133, C164, C292, C323, and/or C347. Preferably, the amino acid sequence of the helicase mutant is as set forth in SEQ ID NO: 6. The helicase mutant exhibits excellent DNA unwinding activity, good protein uniformity, reduced cross-linking interactions between protein molecules, enhanced adaptability with perforin, improved sequencing stability, as well as integrity and continuity of electrical signals. Consequently, it achieves a better sequencing performance. It can be utilized for the control and characterization of nucleic acids and applied in single-molecule nanopore sequencing.

## Description

### FIELD

The present disclosure relates to the field of biotechnology or sequencing technologies, and more particularly, to a helicase mutant, a preparation method therefor, and use thereof in high-throughput sequencing.

### BACKGROUND

As an emerging single-molecule sequencing technology, nanopore sequencing technology has brought disruptive changes to the gene sequencing industry with its unique advantages such as high throughput, long read lengths, high speed, in situ detection, and label-free operation. This technology does not require imaging equipment for detection, enabling the system to be reduced to a portable level, thus meeting different sequencing scenarios. Also, due to its feature of direct sequencing without amplification, nanopore sequencing technology has no restrictions on the length of the DNA to be sequenced. It allows for real-time base calling, and can achieve direct sequencing of modifications such as RNA and methylation, as well as other single molecules. Nanopore sequencing technology has a wide range of applications in both basic theoretical research in life sciences and in biomedical clinical practice.

Nanopore sequencing is a sequencing technology based on electrical signals. Double-stranded DNA (dsDNA) is unwound into single-stranded DNA (ssDNA) by DNA helicase. Under the action of an electric field force, the ssDNA passes through the porin anchored on a biological membrane or an artificial membrane. When the ssDNA passes through the nanopore, the magnitudes of current obstructions vary due to different bases. By detecting the current fluctuation signal of the nanopore and analyzing the current signal through machine learning, the DNA sequence of the ssDNA can be determined.

During the sequencing process, due to the extremely high speeds at which nucleic acid molecules pass through the nanopore channel, it is unable to accurately obtain polynucleotide sequence information. Therefore, effectively reducing and controlling the nanopore translocation motion of nucleic acid molecules is a key technical issue in realizing nanopore sequencing. At present, the most common and effective method is to control the nanopore translocation motion of nucleic acid molecules using the idea of helicase-mediated unwinding, thus improving detection accuracy. In addition, to better maintain sequencing speeds and sequencing uniformity, specific modification and improvements of the helicase are urgently needed to improve its control over the movement of polynucleotides and to enhance the stability of nanopore sequencing applications.

### SUMMARY

The helicase used in current commercial nanopore sequencers is the DDA helicase derived from bacteriophage T4, which suffers from a very low yield and a limited application range. A helicase BCH105 (Patent No. PCT/CN2021/143662), whose gene is derived from the deep-sea metagenome, exhibits very high thermal stability and salt tolerance. BCH105 can be highly expressed in an *Escherichia coli* recombinant protein expression system with a very high yield. In addition, BCH105 has a special pin structure, which enables it to show satisfactory single-stranded DNA binding and double-stranded DNA unwinding activities. Therefore, BCH105 can be used for the control and characterization of nucleic acids and applied to single-molecule nanopore sequencing. However, BCH105 has some intermolecular cross-links. By mutational modification of certain key sites to change the cysteine on its surface, the problem of intermolecular cross-linking interactions of its proteins can be mitigated. In addition, during application, BCH105 can interact with perforin, which has a certain impact on the sequencing signal and sequencing speed. By mutating the amino acids at some key sites and modifying the interface where the helicase may interact with the perforin, the interaction of the helicase with DNA or the perforin can be increased, facilitating the formation of a stable complex with the DNA. This make BCH105 more compatible with the perforin, enabling it to achieve better sequencing performance. Moreover, by modifying the pin domain and the tower domain of the helicase, the electrical signal integrity rate can be improved. In summary, there is substantial room for improvement in the protein homogeneity of BCH105, as well as the continuity, stability and integrity of sequencing. Therefore, the present disclosure provides a helicase mutant, a preparation method therefor, and use thereof in high-throughput sequencing.

Specifically, in a first aspect, the present disclosure provides a helicase mutant. The helicase mutant has at least one cysteine on a surface of a three-dimensional structure of an amino acid sequence as set forth in SEQ ID NO: 1, the at least one cysteine being substituted with alanine. Thus, the cross-linking between protein molecules is reduced.

The cysteine is C133, C164, C292, C323, and/or C347. Preferably, the amino acid sequence of the helicase mutant is as set forth in SEQ ID NO: 6.

The helicase mutant further has at least one amino acid mutation in a pin domain, a tower domain, fragment V66-N84, and/or fragment K294-A321. The at least one amino acid mutation is substitution of an original amino acid with cysteine or an unnatural amino acid and/or cross-linking an original amino acid with at least one chemical cross-linking agent. Thus, the cross-linking efficiency is improved and then the sequencing performance is enhanced.

Preferably, a mutation site in the pin domain is at least one of 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, and 134; a mutation site in the tower domain is at least one of 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, and 393; a mutation site in the fragment V66-N84 is at least one of 66, 67, 68, 69, 70, 71, 72, 73, 74, 75, 76, 77, 78, 79, 80, 81, 82, 83, and 84; and/or a mutation site in the fragment K294-A321 is at least one of 294, 295, 296, 297, 298, 299, 300, 301, 302, 303, 304, 305, 306, 307, 308, 309, 310, 311, 312, 313, 314, 315, 316, 317, 318, 319, 320, and 321.

More preferably, the mutation site in the pin domain is at least one of G96, T97, I98, H99, H100, F101, L102, N103, L104, K105, L106, D107, H108, G109, F110, A111, D112, D113, G114, T115, A116, D117, N118, V119, T120, T121, K122, A123, K124, L125, V126, V127, N128, K129, F130, N131, E132, A133, and L134; the mutation site in the tower domain is at least one of P355, S356, S357, Y358, N359, E360, F361, N362, D363, L364, L365, D366, K367, Y368, L369, A370, D371, A372, K373, I374, A375, K376, G377, Y378, D379, R380, S381, K382, A383, W384, K385, K386, Y387, F388, K389, L390, K391, E392, and K39; the mutation site in the fragment V66-N84 is at least one of V66, T67, S68, P69, T70, H71, K72, A73, V74, R75, V76, S78, L79, N80, M81, L82, K83, and N84; and/or the mutation site in the fragment K294-A321 is at least one of K294, D295, E296, L297, V298, F299, Q300, E301, T302, Y303, T304, D305, S306, K307, G308, N309, I310, I311, V312, S313, N314, G315, E316, 1317, I318, E319, V320, and A321.

The unnatural amino acid is selected from the group consisting of 4-azido-L-phenylalanine (PAZF), 4-azido-L-phenylalanine hydrochloride (PAZF-Hcl), 4-acetyl-L-phenylalanine, 3-acetyl-L-phenylalanine, 4-acetoacetyl-L-phenylalanine, O-allyl-L-tyrosine, 3-(phenylselanyl)-L-alanine, O-2-propyn-1-yl-L-tyrosine, 4-(dihydroxyboranyl)-L-phenylalanine, 4-[(ethylthio)carbonyl]-L-phenylalanine, (2S)-2-amino-3-{4-[(propan-2-ylthio)carbonyl]phenyl}propanoic acid, (2S)-2-amino-3-{4-[(2-amino-3-thiopropionyl)amino]phenyl}propanoic acid, O-methyl-L-tyrosine, 4-amino-L-phenylalanine, 4-cyano-L-phenylalanine, 3-cyano-L-phenylalanine, 4-fluoro-L-phenylalanine, 4-iodo-L-phenylalanine, 4-bromo-L-phenylalanine, O-(trifluoromethyl)tyrosine, 4-nitro-L-phenylalanine, 3-hydroxy-L-tyrosine, 3-amino-L-tyrosine, 3-iodo-L-tyrosine, 4-isopropyl-L-phenylalanine, 3-(2-naphthyl)-L-alanine, 4-phenyl-L-phenylalanine, (2S)-2-amino-3-(naphthyl-2-ylamino)propionic acid, 6-(methylsulfanyl)norleucine, 6-oxy-L-lysine, D-tyrosine, (2R)-2-hydroxy-3-(4-hydroxyphenyl)propionic acid, (2R)-2-aminooctanoate 3-(2,2'-bipyridin-5-yl)-D-alanine, 2-amino-3-(8-hydroxy-3-quinolyl)propionic acid, 4-benzoyl-L-phenylalanine, S-(2-nitrobenzyl)cysteine, (2R)-2-amino-3-[(2-nitrobenzyl)thio]propionic acid, (2S)-2-amino-3-[(2-nitrobenzyl)oxy]propionic acid, O-(4,5-dimethoxy-2-nitrobenzyl)-L-serine, (2S)-2-amino-6-({[(2-nitrobenzyl)oxy]carbonyl}amino)hexanoic acid, O-(2-nitrobenzyl)-L-tyrosine, and 2-nitrophenylalanine; and/or the chemical cross-linking agent is selected from the group consisting of maleimide, active ester, succinimide, azide, alkyne, phosgene-type reagent, sulfonyl chloride reagent, isothiocyanate, acyl halide, hydrazine, disulfide, vinyl sulfone, aziridine, and photosensitizer.

One or more amino acid residues at the N-terminus of the amino acid sequence of the helicase mutant as set forth in SEQ ID NO: 6 are truncated; for example, amino acids M1 to A7 are truncated. The helicase mutant is still stable in a monomeric state.

The helicase mutant has at least one amino acid mutation at K2, D4, L5, K82, N83, N84, G85, I86, and D87 of the amino acid sequence as set forth in SEQ ID NO: 6. The at least one amino acid mutation is substitution of an original amino acid with a different amino acid. Preferably the different amino acid is alanine. The helicase mutant is still stable in the monomeric state.

The helicase mutant has at least one mutation in a region interacting with a porin interface. The at least one mutation includes substitution of an original amino acid with alanine, serine, glutamine, leucine, threonine, histidine, or glycine. The helicase mutant still retains the ability to control movement of polynucleotides and can be applied to nanopore sequencing technology.

Preferably, the at least one mutation in the region interacting with the porin interface is at at least one of positions 1, 2, 3, 4, 5, 12, 14, 16, 18, 21, 28, 63, 82, 124, 129, 158, 165, 189, 194, 196, 199, 202, 203, 205, 206, 207, 212, 214, 215, 218, 223, 244, 247, 261, 263, 271, 281, 289, 294, 307, 324, 331, 333, 336, 340, 341, 349, 367, 376, 382, 385, 386, 389, and 393.

More preferably, the at least one mutation in the region interacting with the porin interface is at at least one of positions M1, K2, H3, D4, L5, Q12, Y14, F16, D18, K21, K28, K63, K82, K124, K129, K158, K165, K189, H194, K196, K199, R202, Q203, E205, D206, N207, K212, Q214, E215, K218, K223, K244, K247, K261, K263, K271, R281, K289, K294, K307, K324, K331, K333, K336, K340, K341, R349, K367, K376, K382, K385, K386, K389, and K393.

The helicase mutant has at least one mutation in an ATP hydrolysis region. The at least one mutation includes substitution of an original amino acid with a different charged amino acid or an amino acid with a relatively small side chain. Preferably the different charged amino acid or the amino acid with a relatively small side chain is alanine, aspartic acid, glutamine, or glycine. Thus, the unwinding activity of the helicase mutant is improved, enhancing the sequencing performance, and can be applied to the nanopore sequencing technology.

Preferably, the at least one mutation in the ATP hydrolysis region is at at least one of positions 40, 41, 42, 43, 44, 45, 46, 143, 144, 145, 146, 147, 148, 149, 171, 172, 173, 174, 175, 176, 177, 178, 179, 180, 181, 182, 183, 184, 185, 186, 197, 198, 199, 200, 201, 202, 203, 204, 205, 206, 207, 208, 209, 210, 211, 212, 213, 214, 215, 216, 217, 218, 219, 220, 221, 222, 223, 405, 406, 407, 408, 409, 410, 411, 412, 413, 414, 430, 431, 432, 433, 434, 435, 436, 437, 438, 439, 440, 441, 442, 443, 444, and 445.

More preferably, the at least one mutation in the ATP hydrolysis region is at at least one of positions G40, F41, A42, G43, S44, G45, K46, E144, A145, S146, M147, V148, S149, G171, D172, S173, Y174, Q175, L176, L177, P178, V179, D180, D181, E182, D183, S184, S185, I186, L197, T198, K199, V200, V201, R202, Q203,A204, E205, D206, N207, I208, I209, I210, A211, S213, Q214, E215, L216, I217, K218, A219, M220, D221, Q222, K223, I405, H406, K407, L408, Q409, G410, S411, T412, Y413, Q414, N430, R431, D432, N433, V434, L435, R436, L437, V438, Y439, V440, G441, I442, T443, R444, and A445.

The helicase mutant has a double substitution on the amino acid sequence set forth in SEQ ID NO: 6, selected from the group consisting of:
L369C and D117C,
L369C and V119C,
L369C and D113C,
K373C and G114C,
K373C and D112C,
W384C and F110C,
W384C and D112C,
W384C and G114C,
L369C and T115C,
L369C and N118C,
K373C and T115C,
K373C and A116C,
K373C and D117C,
K373C and N118C,
K373C and V119C,
H108C and F388C,
V119C and W384C,
R380C and A116C,
K373C and T120C,
L369C and T121C,
A116C and 381C,
T121C and K373C,
T120C and L369C, and
T121C and 388C.

Preferably, in the case of the double substitution being L369C and V119C, the helicase mutant further has a mutation selected from:
(1) single substitution selected from the group consisting of K21S, K28S, K189S, H194S, K196S, K199S, Q203S, N207S, K212S, Q214S, K218S, K223S, R202K, E215A, K2A, H3A, D4A, L5A, Y14A, F16A, D18A, K63A, K82A, K245A, K247A, K261A, R281A, K307A, K324A, K331A, K333A, K336A, K341A, K349A, K124A, K129A, K158A, K271A, K376A, K382A, K385A, K393A, and E205D; or
(2) double substitution of K72C and G315C or double substitution of E205A and D206A; or
(3) truncation of 1-st to 7-th amino acids; or
(4) truncation of M1G and an H before the amino acid sequence.

In a second aspect, the present disclosure provides an isolated nucleic acid, which encodes the helicase mutant in the first aspect of the present disclosure.

Preferably, the nucleotide sequence of the helicase mutant is as set forth in SEQ ID NO: 8.

In a third aspect, the present disclosure provides a recombinant expression vector including a promoter and the nucleic acid in the second aspect of the present disclosure.

Preferably, the promoter is T7; and/or a backbone plasmid of the recombinant expression vector is PET.28a(+), PET.21a(+), or PET.32a(+).

In a fourth aspect, the present disclosure provides a transformant including a host cell and the nucleic acid in the second aspect of the present disclosure.

Preferably, the host cell is *Escherichia coli,* more preferably BL21 (DE3), BL21 Star (DE3) pLyss, Rossata (DE3), or Lemo21 (DE3).

In a fifth aspect, the present disclosure provides a method for preparing the helicase mutant in the first aspect of the present disclosure. The method includes culturing the transformant in the fourth aspect of the present disclosure in a culture medium to ferment and produce the helicase mutant.

In a sixth aspect, the present disclosure provides a helicase mutant-sequencing adapter complex including the helicase mutant in the first aspect of the present disclosure and a sequencing adapter.

In a seventh aspect, the present disclosure provides a kit. The kit includes the helicase mutant in the first aspect of the present disclosure and/or the helicase mutant-sequencing adapter complex in the sixth aspect of the present disclosure, and preferably further includes a single-stranded DNA with an anchored molecule at a 5' end, a nanopore, a nanopore protein, an electrical signal detector, a membrane, and/or a buffer.

Preferably, the anchored molecule is a hydrophobic molecule, preferably selected from any one or more of: lipid, fatty acid, sterol, carbon nanotube, polypeptide, protein, and/or amino acid, such as cholesterol, palmitate or tocopherol.

The nanopore is a transmembrane protein pore or a solid pore, preferably selected from the group consisting of hemolysin, MspA, MspB, MspC, MspD, FraC, ClyA, PA63, CsgG, CsgD, XcpQ, SP1, phi29 connector, InvG, GspD, and a combination thereof.

The membrane is selected from the group consisting of an amphiphilic membrane (e.g., a phospholipid bilayer), a high molecular polymer membrane (e.g., a di-block copolymer or a tri-block copolymer), and a combination thereof.

The buffer is selected from the group consisting of a dihydrogen phosphate-hydrogen phosphate buffer system, a carbonic acid-sodium bicarbonate buffer system, a Tris-HCl buffer system, a HEPES buffer system, a MOPS buffer system, and a combination thereof.

In an eighth aspect, the present disclosure provides use of the helicase mutant in the first aspect of the present disclosure, the helicase mutant-sequencing adapter complex in the sixth aspect of the present disclosure, or the kit in the seventh aspect of the present disclosure in high-throughput sequencing.

Preferably, the high-throughput sequencing is nanopore sequencing.

In a ninth aspect, the present disclosure provides a DNA unwinding method. The method includes unwinding double-stranded DNA using the helicase mutant in the first aspect of the present disclosure, the helicase mutant-sequencing adapter complex in the sixth aspect of the present disclosure, or the kit in the seventh aspect of the present disclosure.

In a tenth aspect, the present disclosure provides a sequencing method. The method includes unwinding DNA using the DNA unwinding method in the ninth aspect of the present disclosure and simultaneously sequencing.

Beneficial effects of the technical solution of the present disclosure:

The present disclosure provides a helicase mutant based on the helicase BCH105, and is named BCH105-1, BCH105-2, BCH105-3, and so on. Due to its excellent DNA unwinding activity and good protein uniformity, the helicase mutant can be used for the control and characterization of nucleic acids and applied to single-molecule nanopore sequencing. In addition, compared with BCH105, the helicase mutant reduces the cross-linking interaction between protein molecules, improves its adaptability with perforin, and enhances the stability of sequencing as well as the integrity and continuity of electrical signals, thereby achieving better sequencing performance.

### BRIEF DESCRIPTION OF THE DRAWINGS

FIG. 1 shows SDS-PAGE patterns of BCH105 (left) and BCH105-1 (right) after protein purification.
FIG. 2 shows SDS-PAGE patterns (10%) of BCH105 (left) and BCH105-1 (right) after incubation with an oxidant.
FIG. 3 shows an SDS-PAGE pattern (10%) of BCH105-2 after protein purification.
FIG. 4 shows the purity analysis of the constructed BCH105-2 complex (DNA Native PAGE/8%).
FIG. 5 is a schematic diagram of an adapter (a: upper strand; b: lower strand).
FIG. 6 is a schematic diagram of a sequencing library containing BCH105 and its mutants (a: upper strand; b: lower strand; c: double-stranded target fragment; d: BCH105 and its mutants; e: chol-ssDNA).
FIG. 7 shows current traces as the BCH105-2 controls the translocation of nucleic acid fragments through a nanopore.
FIG. 8 shows an SDS-PAGE pattern (10%) of a BCH105-3 mutant protein.
FIG. 9 shows the purity analysis of the constructed BCH105-3 complex (DNA Native PAGE/8%).
FIG. 10 shows current traces as the BCH105-3 controls the translocation of nucleic acid fragments through a nanopore.
FIG. 11 shows the electrical signal integrity rate of BCH105 mutants (tested at 28°C).
FIG. 12 shows an SDS-PAGE pattern (10%) of BCH105-33 after protein purification.
FIG. 13 shows the purity analysis of the constructed BCH105-33 complex (DNA Native PAGE/8%).
FIG. 14 shows current traces as the BCH105-33 controls the translocation of nucleic acid fragments through a nanopore.
FIG. 15 is a distribution diagram of the sequencing speed of BCH105-3, BCH105-33 and BCH105-72 (tested at 30°C).
FIG. 16 is a predicted structure diagram of BCH105 by Alphafold2.

### DETAILED DESCRIPTION

### Example 1: Cloning, Expression, and Purification of BCH105 and Its Mutant BCH105-1

### 1. Vector Construction and Expression of BCH105 Helicase and Its Mutant BCH105-1

The amino acid sequence of BCH105 is set forth in SEQ ID NO: 1, and its full-length DNA sequence is set forth in SEQ ID NO: 5. The full-length DNA sequence was synthesized (Liuhe, BGI) and ligated into a PET-28a(+) plasmid using double restriction sites Nde1 and Xho1. Therefore, the expressed BCH105 protein had a His tag and a thrombin restriction site at the N-terminus. Its mutant was constructed by site-directed mutagenesis based on this plasmid.

The cloned PET.28a(+)-BCH105 plasmid and its mutant BCH105-1 were transformed into the *Escherichia coli* expression strain BL21(DE3) or its derivatives. A single colony was picked, inoculated into 5 mL of LB medium containing kanamycin, and cultured at 37°C overnight with shaking. Then it was transferred to 1 L of LB containing kanamycin and cultured at 37°C with shaking until OD600 = 0.6-0.8. After the temperature was lowered to 16°C, IPTG with a final concentration of 500 µM was added to induce expression overnight.

### 2. Purification of BCH105 and Its Mutant BCH105-1

The formulas of the buffers used are as follows:
Buffer A: 20 mM Tris-HCl pH 8.0, 200 mM NaCl, 20 mM imidazole
Buffer B: 20 mM Tris-HCl pH 8.0, 100 mM NaCl, 20 mM imidazole
Buffer C: 20 mM Tris-HCl pH 8.0
Buffer D: 20 mM Tris-HCl pH 8.0, 50 mM NaCl
Buffer E: 20 mM Tris-HCl pH 8.0, 1000 mM NaCl
Buffer F: 20 mM Tris-HCl pH 8.0, 80 mM NaCl

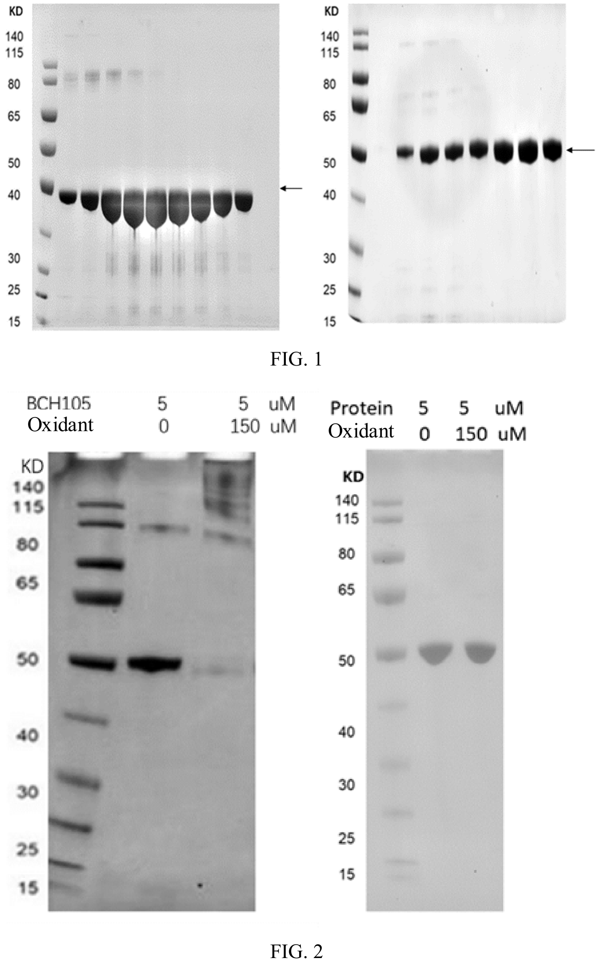

In order to collect the expressed bacterial cells, the bacterial cells expressing BCH105 and its mutant BCH105-1 were centrifuged at a rotational speed of 6500 rpm, resuspended in buffer A, crushed using a cell pressure crusher, and then centrifuged at 18000 rpm for 1 h to obtain the supernatant. The supernatant was mixed with Ni-NTA filler, which had been equilibrated with buffer A, and incubated for 1h. The filler was then collected and washed with buffer B in large quantities until no impure proteins were washed out. Afterward, Thrombin was added to the filler and incubated overnight at 4°C for 12-16 h. The flow-through was collected and diluted into 50 mM NaCl with Buffer C. The flow-through containing the target protein was then passed through a QFF column with Buffer D and subjected to gradient elution with Buffer E. The target protein peak was collected and concentrated. After concentration, the protein was loaded onto a molecular sieve Superdex^{®} 200, and the molecular sieve buffer used was Buffer F. The target protein peak was collected, concentrated, and stored frozen. As illustrated in FIG. 1, after purification, a large amount of mutant protein with satisfactory purity can be finally obtained.

### Example 2: Test on the Intermolecular Aggregation State of BCH105 and Its Mutant BCH105-1

### 1. Determination of the Intermolecular or Intramolecular Aggregation State of BCH105

BCH105 and an oxidant were incubated at a fixed ratio at a constant temperature of 30°C for 0.5 h, with the protein concentration fixed at 5 µM and the oxidant concentration of 0 and 150 µM. After incubation, 10 µL of the sample was subjected to SDS-PAGE (10%). The results are shown in FIG. 2.

As can be seen from FIG. 2 (left), in the absence of an oxidant, there was only one target protein band on the SDS-PAGE. However, after incubation with an oxidant, the protein band shifted upward and multiple bands appeared, indicating that cross-linking interaction occurred between BCH105 proteins. It is speculated that in the presence of an oxidant, the protein underwent intermolecular or intramolecular cross-linking, resulting in protein aggregation and upward movement of bands. This will lead to non-uniformity of such proteins, thus posing potential risks when applied to sequencing.

### 2. Determination of the Intermolecular or Intramolecular Aggregation State of BCH105 and BCH105-1

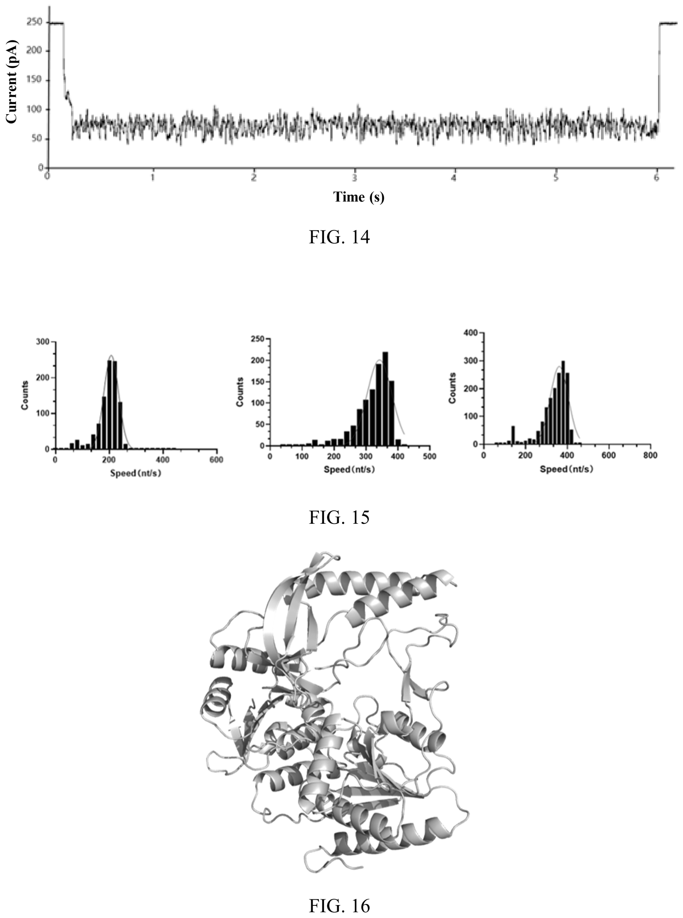

BCH105-1 was designed based on the predicted structure diagram of BCH105 by Alphafold2 (FIG. 16). Five cysteines located on the surface of the protein's three-dimensional structure were all mutated to alanine. The specific mutation site information is shown in Table 1. After protein expression and purification, the protein and an oxidant were mixed at a certain ratio and reacted using the same method as above.

The results are shown in FIG. 2 (right). It can be seen from the figure that the there was only one target protein band foe BCH105-1 protein both without and with the addition of the oxidant. This indicates that after the cysteines on the surface of the protein were mutated to alanine, the intermolecular or intramolecular cross-linking can be significantly reduced, which can increase the protein's uniformity and make it more suitable for nanopore sequencing.

### Example 3: Construction of BCH105 Series Mutant Complexes

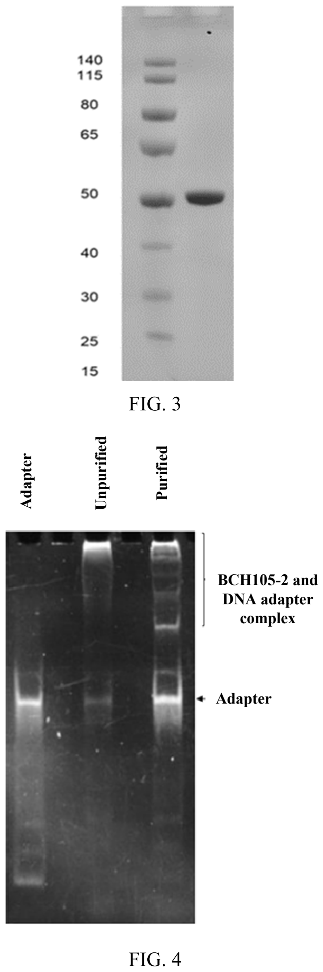

A relatively pure complex formed by helicase and DNA can be better applied to nanopore sequencing technology, improving the stability and continuity of nanopore sequencing. Therefore, constructing a protein-DNA complex is an important step in nanopore sequencing. The purity of the complex (i.e., a ratio of protein to adapter is 1:1) will directly affect the sequencing results. When the ratio of free adapter to protein is N:1, it may cause pore-blocking or other issues that lead to poor sequencing results. A higher purity leads to a better sequencing efficiency. Therefore, improving the purity of the complex is important. To construct a helicase-DNA adapter complex, the protein and the protein-binding base region of the adapter need to be cross-linked and fixed to prevent DNA to be sequenced from detaching from the sequencing region during the sequencing process, which could otherwise result in unstable and discontinuous sequencing. Through the analysis of the helicase structure, the amino acids in the Pin and Tower regions of BCH105-1 were mutated and cysteines were introduced to obtain a mutant BCH105-2 (SEQ ID NO: 6). The specific mutation site information of BCH105-2 is shown in Table 1, and protein results are shown in FIG. 3. The BCH105-2 protein and the DNA adapter were mixed at a certain ratio and incubated at 28°C for 1 h. Then, the protein and a cross-linking agent were added to an oxidative cross-linking agent at a certain ratio and incubated at 28°C for 1.5 h. Finally, ATP with a final concentration of 4 µM was added and incubated at 30°C for 1.5 h. 10 µL of the sample was collected as an unpurified sample for electrophoresis. The remaining sample was purified using magnetic beads to obtain the desired complex. The complex was stored in 20% glycerol in a -80°C refrigerator. A small amount of sample (with the same mass as the unpurified sample) was taken as a purified sample for DNA Native PAGE gel. The results are shown in FIG. 4.

It can be concluded from FIG. 4 that the protein BCH105-2 can form a complex with DNA under the action of the oxidant. However, multiple bands were formed between the protein and DNA. It was identified that the second brightest band was the stable 1:1 complex. The bands above this band were all N:1 bands, and the bands below this band were free DNA adapter bands.

### Example 4: Characterization of BCH105-2 and Its Mutant and Control of Nucleic Acids

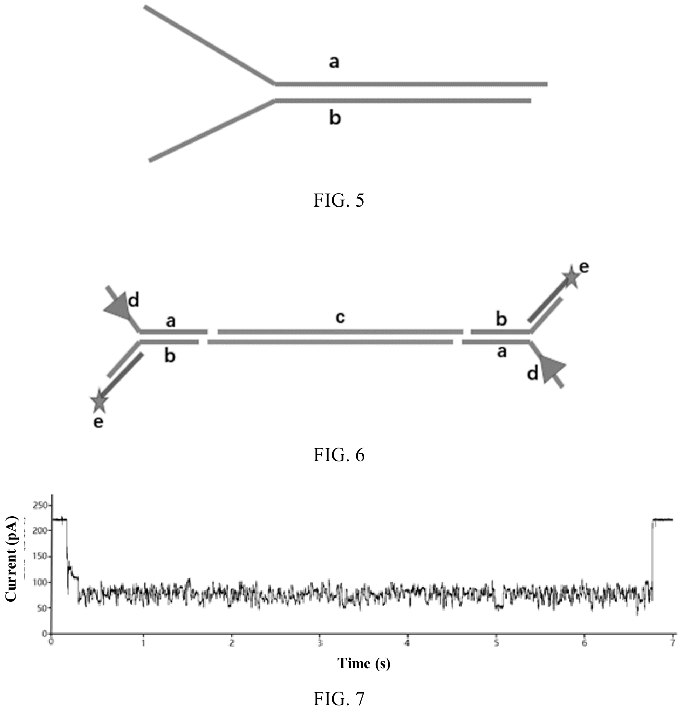

1. Two partially complementary DNA strands (the upper strand, SEQ ID NO: 2 and the lower strand, SEQ ID NO: 3) were annealed to form an adapter, which was ligated with the double-stranded target fragment to be sequenced at room temperature using T4 DNA ligase (NEB, E6057AVIAL) and then purified to prepare a sequencing library.
2. The BCH105-2 protein was incubated with the sequencing library at 25°C for 1 h (a molar concentration ratio of 1:8) to form a sequencing library containing helicase.
3. The sequencing library containing helicase was incubated with single-stranded DNA with cholesterol at the 5' end (ssDNA-chol, SEQ ID NO: 4) at room temperature for 10 min. The ssDNA-chol sequence was complementary to a part of the lower strand of the adapter. After cholesterol bound to the phospholipid membrane, it can reduce the amount of library loaded and increase the capture rate.
4. The current signal was collected using a patch clamp amplifier or other electrical signal amplifier. A Teflon^{™} membrane with micro-sized pores (diameter 50 µm to 200 µm) divided the electrolytic cell into two chambers, the cis-chamber and the trans-chamber. A pair of Ag/AgCl electrodes was placed in each of the cis-chamber and the trans-chamber. After a bilayer phospholipid membrane formed at the micropores of the two chambers, the nanopore protein CsgG-Eco-(Y51A/F56Q/R97TW/R192D-StrepII(C)) was added. After a single nanopore protein inserted into the phospholipid membrane, electrical measurements were obtained. The reaction product from step 3 was added and 180 mV was applied. The sequencing library was captured by the nanopore and the nucleic acids passed through the nanopore under the control of the helicase. The buffer used in this experiment was: 0.47 M KCl, 25 mM HEPES, 1 mM EDTA, 5 mM ATP, 25 mM MgCl₂, pH 7.6. The sequencing temperature was 28°C.
5. A sequencing experiment was performed using BCH105-2. The sequencing electrical signals are shown in FIG. 7. As can be seen from the results, as the helicase controlled the single-stranded DNA to enter the nanopore, part of the current was blocked and the current decreased. Since different nucleotides had different sizes, the magnitudes of the blocked current were also different, in such a manner that a fluctuating current signal can be observed.

### Example 5: Amino Acid Modification and Optimization of the Pin and Tower Domains of BCH105 Series Helicases

To better apply helicases like BCH105 in nanopore sequencing technology, an important sequencing indicator needs to be considered: the sequencing electrical signal integrity rate. The sequencing electrical signal integrity rate refers to the degree to which the electrical signal obtained by sequencing a known sequence with a special electrical signal is recognized by an algorithm and subjected to deep learning to achieve complete recognition of the sequencing signal of the special electrical signal of the known sequence. A higher electrical signal integrity rate recognized by the algorithm indicates the better sequencing effect. Through in-depth analysis of helicase in nanopore sequencing technology, the Pin and Tower domains were found to have a certain influence on this performance. Since BCH105-1, obtained by mutating the cysteines on the surface of the BCH105 protein structure to alanine, has no intermolecular cross-linking, it can be better applied in nanopore sequencing. Therefore, the amino acids in the Pin and Tower domains were modified to introduce one or more cysteines for cross-linking, hoping to further improve the application of BCH105 series helicases in nanopore sequencing technology by changing the sites in these regions.

The plasmids were constructed by site-directed mutagenesis to obtain mutants BCH105-3 to BCH105-26. The mutation information is shown in Table 1. The subsequent methods for mutant protein expression, purification, and sequencing were the same as described above.

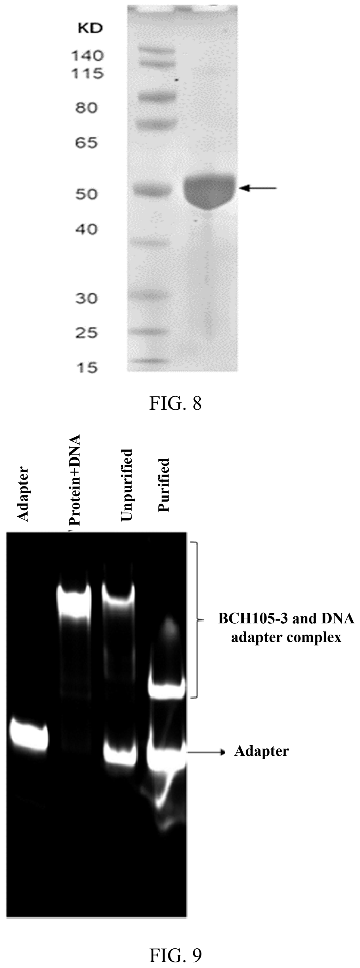

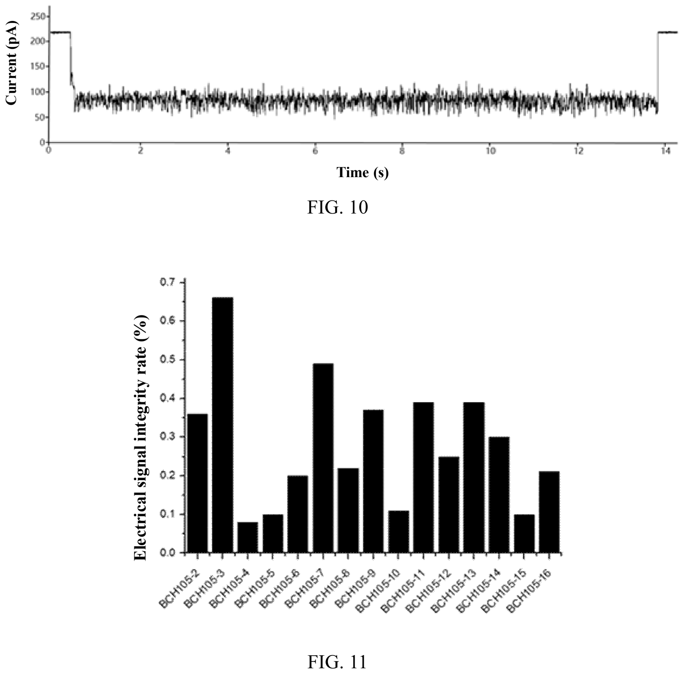

The experimental results are shown in FIG. 8 to FIG. 10. As can be seen from FIG. 8, the protein exhibited a high purity. By comparing FIG. 9 and FIG. 4, the complex purity (i.e., a ratio of a protein to DNA is 1:1) of BCH105-3 mutant was higher than that of BCH105-2. After constructing a library with this complex and conducting an on-machine test, the current changes of the nucleic acid fragment to be sequenced for BCH105-3 is shown in FIG. 10. The sequencing electrical signal integrity rate was significantly improved, indicating that modifying the amino acids in the Pin and Tower domains can effectively improve the electrical signal integrity rate of BCH105-type helicases and optimize their performance in nanopore sequencing technology.

### Example 6: Amino Acid Modification and Optimization of the Potential Interaction Interfaces between BCH105 Series Helicases and Porin

To better apply helicases like BCH105 in nanopore sequencing technology, more performance indicators of BCH105 in nanopore sequencing technology need to be considered for evaluation, such as the electrical signal capture rate and the sequencing speed. Higher sequencing speed and higher electrical signal capture rate indicate greater sequencing throughput. During the sequencing process, helicase functions by interacting with porins and DNA, which can increase the adaptability between helicases and porins or DNA, and thus improve sequencing performance. Optimization of the Pin and Tower domains improved the efficiency and purity of forming a 1:1 complex during the complex construction process, and thus improved sequencing performance. After analyzing the sequencing results, BCH105-3 was selected as having a good improvement in the performance of capturing a complete electrical signal. Therefore, based on BCH105-3, certain key sites that may interact with the perforin interface were modified, mainly focusing on mutation of the amino acids in the 1A/2A/2B regions. The mutation mainly included replacing positively charged amino acids with neutrally charged amino acids or negatively charged amino acids, and/or replacing long side chain amino acids with short side chain amino acids, so as to increase the adaptability between BCH105-type helicases and porins and optimize certain key sequencing indicators.

The plasmids were constructed by site-directed mutagenesis to obtain mutants BCH105-27 to BCH105-73. The mutation information is shown in Table 1. The subsequent methods for mutant protein expression, purification, and sequencing were the same as described above.

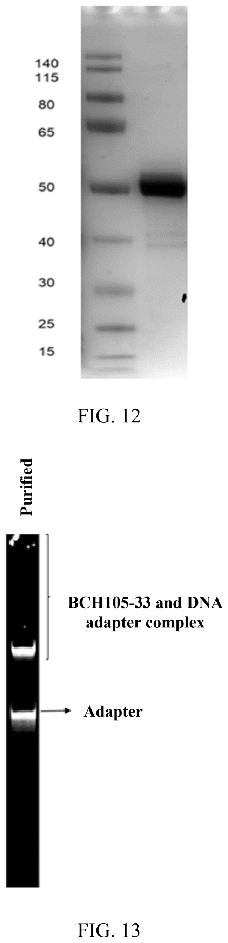

The sequencing results are shown in FIG. 12 to FIG. 14.

As illustrated in FIG. 15, the speeds of both BCH105-33 and BCH105-72 were higher than that of BCH105-3. This indicates that the 1A/2A/2B domains of BCH105-type helicases interact with porin during the sequencing process, and the mutation directions of amino acids are also relatively clear.

[

**Table 1] Mutation Site Information of BCH105 Mutants**

| Name | Cys mutation on the three-dimensional structure surface | Mutation in Pin domain and/or Tower domain | Mutation in the region interacting with porin interface |
|---|---|---|---|
| BCH105 | / | / | / |
| BCH105-1 | C133A/C164A/C292A/C323A/C347A | | |
| BCH105-2 | C133A/C164A/C292A/C323A/C347A | D117C+L369C | |
| BCH105-3 | C133A/C164A/C292A/C323A/C347A | L369C+V119C | |
| BCH105-4 | C133A/C164A/C292A/C323A/C347A | L369C+D113C | |
| BCH105-5 | C133A/C164A/C292A/C323A/C347A | K373C+G114C | |
| BCH105-6 | C133A/C164A/C292A/C323A/C347A | K373C+D112C | |
| BCH105-7 | C133A/C164A/C292A/C323A/C347A | W384C+F110C | |
| BCH105-8 | C133A/C164A/C292A/C323A/C347A | W384C+D112C | |
| BCH105-9 | C133A/C164A/C292A/C323A/C347A | W384C+G114C | |
| BCH105-10 | C133A/C164A/C292A/C323A/C347A | L369C+T115C | |
| BCH105-11 | C133A/C164A/C292A/C323A/C347A | L369C+N118C | |
| BCH105-12 | C133A/C164A/C292A/C323A/C347A | K373C+T115C | |
| BCH105-13 | C133A/C164A/C292A/C323A/C347A | K373C+A116C | |
| BCH105-14 | C133A/C164A/C292A/C323A/C347A | K373C+D117C | |
| BCH105-15 | C133A/C164A/C292A/C323A/C347A | K373C+N118C | |
| BCH105-16 | C133A/C164A/C292A/C323A/C347A | K373C+V119C | |
| BCH105-17 | C133A/C164A/C292A/C323A/C347A | H108C+F388C | |
| BCH105-18 | C133A/C164A/C292A/C323A/C347A | V119C+W384C | |
| BCH105-19 | C133A/C164A/C292A/C323A/C347A | R380C+A116C | |
| BCH105-20 | C133A/C164A/C292A/C323A/C347A | K373C+T120C | |
| BCH105-21 | C133A/C164A/C292A/C323A/C347A | L369C+T121C | |
| BCH105-22 | C133A/C164A/C292A/C323A/C347A | A116C+381C | |
| BCH105-23 | C133A/C164A/C292A/C323A/C347A | T121C+K373C | |
| BCH105-24 | C133A/C164A/C292A/C323A/C347A | T120C+L369C | |
| BCH105-25 | C133A/C164A/C292A/C323A/C347A | T121C+388C | |
| BCH105-26 | C133A/C164A/C292A/C323A/C347A | L369C+V119C | K72C+G315C |
| BCH105-27 | C133A/C164A/C292A/C323A/C347A | L369C+V119C | Truncated M1-A7 |
| BCH105-28 | C133A/C164A/C292A/C323A/C347A | L369C+V119C | K21S |
| BCH105-29 | C133A/C164A/C292A/C323A/C347A | L369C+V119C | K28S |
| BCH105-30 | C133A/C164A/C292A/C323A/C347A | L369C+V119C | K189S |
| BCH105-31 | C133A/C164A/C292A/C323A/C347A | L369C+V119C | H194S |
| BCH105-32 | C133A/C164A/C292A/C323A/C347A | L369C+V119C | K196S |
| BCH105-33 | C133A/C164A/C292A/C323A/C347A | L369C+V119C | K199S |
| BCH105-34 | C133A/C164A/C292A/C323A/C347A | L369C+V119C | Q203S |
| BCH105-35 | C133A/C164A/C292A/C323A/C347A | L369C+V119C | N207S |
| BCH105-36 | C133A/C164A/C292A/C323A/C347A | L369C+V119C | K212S |
| BCH105-37 | C133A/C164A/C292A/C323A/C347A | L369C+V119C | Q214S |
| BCH105-38 | C133A/C164A/C292A/C323A/C347A | L369C+V119C | K218S |
| BCH105-39 | C133A/C164A/C292A/C323A/C347A | L369C+V119C | K223S |
| BCH105-40 | C133A/C164A/C292A/C323A/C347A | L369C+V119C | R202K |
| BCH105-41 | C133A/C164A/C292A/C323A/C347A | L369C+V119C | E215A |
| BCH105-42 | C133A/C164A/C292A/C323A/C347A | L369C+V119C | M1G+ (an H deleted preceding the protein sequence) |
| BCH105-43 | C133A/C164A/C292A/C323A/C347A | L369C+V119C | K2A |
| BCH105-44 | C133A/C164A/C292A/C323A/C347A | L369C+V119C | H3A |
| BCH105-45 | C133A/C164A/C292A/C323A/C347A | L369C+V119C | D4A |
| BCH105-46 | C133A/C164A/C292A/C323A/C347A | L369C+V119C | L5A |
| BCH105-47 | C133A/C164A/C292A/C323A/C347A | L369C+V119C | Y14A |
| BCH105-48 | C133A/C164A/C292A/C323A/C347A | L369C+V119C | F16A |
| BCH105-49 | C133A/C164A/C292A/C323A/C347A | L369C+V119C | D18A |
| BCH105-50 | C133A/C164A/C292A/C323A/C347A | L369C+V119C | K63A |
| BCH105-51 | C133A/C164A/C292A/C323A/C347A | L369C+V119C | K82A |
| BCH105-52 | C133A/C164A/C292A/C323A/C347A | L369C+V119C | K165A |
| BCH105-53 | C133A/C164A/C292A/C323A/C347A | L369C+V119C | K244A |
| BCH105-54 | C133A/C164A/C292A/C323A/C347A | L369C+V119C | K247A |
| BCH105-55 | C133A/C164A/C292A/C323A/C347A | L369C+V119C | K261A |
| BCH105-56 | C133A/C164A/C292A/C323A/C347A | L369C+V119C | R281A |
| BCH105-57 | C133A/C164A/C292A/C323A/C347A | L369C+V119C | K307A |
| BCH105-58 | C133A/C164A/C292A/C323A/C347A | L369C+V119C | K324A |
| BCH105-59 | C133A/C164A/C292A/C323A/C347A | L369C+V119C | K331A |
| BCH105-60 | C133A/C164A/C292A/C323A/C347A | L369C+V119C | K333A |
| BCH105-61 | C133A/C164A/C292A/C323A/C347A | L369C+V119C | K336A |
| BCH105-62 | C133A/C164A/C292A/C323A/C347A | L369C+V119C | K341A |
| BCH105-63 | C133A/C164A/C292A/C323A/C347A | L369C+V119C | R349A |
| BCH105-64 | C133A/C164A/C292A/C323A/C347A | L369C+V119C | K124A |
| BCH105-65 | C133A/C164A/C292A/C323A/C347A | L369C+V119C | K129A |
| BCH105-66 | C133A/C164A/C292A/C323A/C347A | L369C+V119C | K158A |
| BCH105-67 | C133A/C164A/C292A/C323A/C347A | L369C+V119C | K271A |
| BCH105-68 | C133A/C164A/C292A/C323A/C347A | L369C+V119C | K376A |
| BCH105-69 | C133A/C164A/C292A/C323A/C347A | L369C+V119C | K382A |
| BCH105-70 | C133A/C164A/C292A/C323A/C347A | L369C+V119C | K385A |
| BCH105-71 | C133A/C164A/C292A/C323A/C347A | L369C+V119C | K393A |
| BCH105-72 | C133A/C164A/C292A/C323A/C347A | L369C+V119C | E205D |
| BCH105-73 | C133A/C164A/C292A/C323A/C347A | L369C+V119C | E205A+D206A |

**[Table 2] Various Indicators of BCH105 Mutants in Nanopore Sequencing Technology**

| Name | Speed (nt/s) | Single-pore Channel Capture Number | Integrity Rate |
|---|---|---|---|
| BCH105-2 | 210 | 44 | 36% |
| BCH105-3 | 245 | 255 | 66% |
| BCH105-4 | 237 | 183 | 8% |
| BCH105-5 | 238 | 218 | 10% |
| BCH105-6 | 199 | 88 | 20% |
| BCH105-7 | 232 | 169 | 49% |
| BCH105-8 | 246 | 135 | 22% |
| BCH105-9 | 132 | 19 | 37% |
| BCH105-10 | 230 | 44 | 11% |
| BCH105-11 | 232 | 143 | 39% |
| BCH105-12 | 240 | 92 | 25% |
| BCH105-13 | 245 | 196 | 39% |
| BCH105-14 | 238 | 12 | 30% |
| BCH105-15 | 240 | 244 | 10% |
| BCH105-16 | 236 | 332 | 21% |
| BCH105-17 | 340 | 76 | 50% |
| BCH105-18 | 327 | 135 | 32% |
| BCH105-19 | 295 | 325 | 1% |
| BCH105-20 | 316 | 108 | 11% |
| BCH105-21 | 375 | 30 | 19% |
| BCH105-22 | 365 | 104 | 23% |
| BCH105-23 | 320 | 23 | 30% |
| BCH105-24 | 351 | 298 | 53% |
| BCH105-25 | 331 | 16 | 46% |
| BCH105-26 | 338 | 184 | 46% |
| BCH105-27 | 216 | 310 | 62% |
| BCH105-28 | 342 | 41 | 58% |
| BCH105-29 | 345 | 340 | 67% |
| BCH105-30 | 304 | 315 | 75% |
| BCH105-31 | 300 | 316 | 73% |
| BCH105-32 | 313 | 314 | 68% |
| BCH105-33 | 358 | 270.5 | 63% |
| BCH105-34 | 236 | 243 | 69% |
| BCH105-35 | 343 | 199 | 63% |
| BCH105-36 | 270 | 75.5 | 64% |
| BCH105-37 | 207 | 71 | 68% |
| BCH105-38 | 336 | 115.5 | 63% |
| BCH105-39 | 343 | 208 | 63% |
| BCH105-40 | 179 | 178 | 69% |
| BCH105-41 | 336 | 207 | 63% |
| BCH105-42 | 363 | 169.5 | 62% |
| BCH105-43 | 327 | 166 | 62% |
| BCH105-44 | 330 | 91.5 | 67% |
| BCH105-45 | 332 | 72 | 63% |
| BCH105-46 | 300 | 115 | 57% |
| BCH105-47 | 318 | 112.6 | 68% |
| BCH105-48 | 250 | 44 | 66% |
| BCH105-49 | 324 | 143 | 65% |
| BCH105-50 | 323 | 76.5 | 64% |
| BCH105-51 | 360 | 157 | 51% |
| BCH105-52 | 345 | 138.5 | 45% |
| BCH105-53 | 349 | 53 | 73% |
| BCH105-54 | 332 | 13 | 64% |
| BCH105-55 | 360 | 82.5 | 37% |
| BCH105-56 | 360 | 88.5 | 70% |
| BCH105-57 | 349 | 40 | 63% |
| BCH105-58 | 348 | 56.5 | 39% |
| BCH105-59 | 348 | 107 | 44% |
| BCH105-60 | 350 | 26 | 76% |
| BCH105-61 | 362 | 82.5 | 68% |
| BCH105-62 | 362 | 30 | 72% |
| BCH105-63 | 340 | 67.5 | 67% |
| BCH105-64 | 344 | 68 | 63% |
| BCH105-65 | 360 | 66.5 | 58% |
| BCH105-66 | 330 | 62.5 | 61% |
| BCH105-67 | 355 | 89 | 52% |
| BCH105-68 | 355 | 51 | 69% |
| BCH105-69 | 350 | 98.5 | 62% |
| BCH105-70 | 353 | 27 | 72% |
| BCH105-71 | 360 | 82.5 | 71% |
| BCH105-72 | 365 | 76.5 | 56% |
| BCH105-73 | 355nt/s | 153 | 62% |

The sequences used in the present disclosure are as follows:
Amino acid sequence of BCH105 (SEQ ID NO: 1): 
MKHDLSSATEEQRYIFTDFIKQAQLALKGDSDYEVFAIQGFAGSGKTWLSA
LIIDELLELGMKVAVTSPTHKAVRVSLNMLKNNGIDTNSPLMYPGTIHHFLNLKLDHG
FADDGTADNVTTKAKLVVNKFNECLEYVDVLIVDEASMVSGELYDHALKTLGDRCK
IILFIGDSYQLLPVDDEDSSIFLKDDIFHYKLTKVVRQAEDNIIIAKSQELIKAMDQKTY
YPSVNDYFVNITEDTEGIKLLKSNVELFELYFSDFKDKMTGAYTNKVVNQFNEYIRYT
LYQETKFICDKDELVFQETYTDSKGNIIVSNGEIIEVATCKLTTDIDKFKIWKIVSKKNEL
GECVRFNVLDPSSYNEFNDLLDKYLADAKIAKGYDRSKAWKKYFKLKEKYAKVRYN
 FSSTIHKLQGSTYQNMYFDMRGLDYFYRMNRDNVLRLVYVGITRASDQVFILQD
SEQ ID NO: 2:
   5'-TTTTTTTTTTTTTTTTTTTTTTTTTTTTTTTTTTTTTTTT-
   YYYYGGTTGTTTCTGTTGGTGCTGATATTGCT-3' (Y = iSp18)
   where iSp18 is illustrated in the following formula I: 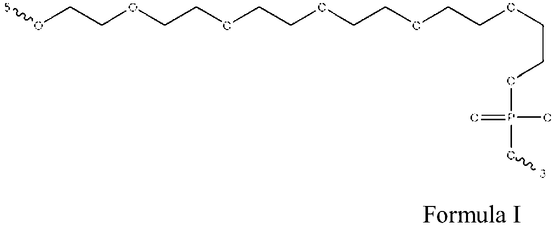
SEQ ID NO: 3:
   5'-GCAATATCAGCACCAACAGAAACAACCTTTGAGGCGAGCGGTCAA-3'
SEQ ID NO: 4:
   5'-cholesterol-TTGACCGCTCGCCTC-3'
DNA sequence corresponding to BCH105 protein (SEQ ID NO: 5) 
atgaaacatgatctgagcagcgcaaccgaagaacagcgttatatttttaccgatttcatcaagcaggcccagctggcac
tgaaaggtgatagcgattatgaagtttttgccatccagggttttgccggtagcggtaaaacctggctgagcgcactgattattgatgaact
gctggaactgggtatgaaagttgcagttaccagcccgacccataaagcagttcgtgttagcctgaatatgctgaaaaataacggcattga
caccaacagcccgctgatgtatccgggtaccattcatcattttctgaatctgaaactggaccacggttttgccgatgatggtaccgcagat
aatgttaccaccaaagcaaaactggttgtgaacaagttcaacgagtgtctggaatacgttgacgttctgatcgttgatgaagcaagcatg
gttagcggtgaactgtatgatcatgcactgaaaaccctgggtgatcgttgtaaaattattctgtttatcggcgacagctaccagctgctgcc
ggttgatgatgaagatagcagcatttttctgaaggacgacatttttcactacaagctgaccaaagtggtgcgccaggcagaagataatatt
attattgccaaaagccaggagctgatcaaggcaatggatcagaaaacctattacccgagcgttaatgactatttcgttaacatcaccgag
gacaccgaaggcattaaactgctgaaaagcaatgttgagctgttcgaactgtactttagcgacttcaaggataagatgaccggcgcatat
accaataaagttgtgaatcagtttaacgagtacatccgctataccctgtatcaggaaaccaaatttatttgcgataaggacgagctggtgtt
ccaggaaacctataccgatagcaaaggtaatattattgtgagcaatggcgagatcatcgaggttgcaacctgtaaactgaccaccgatat
tgataaattcaagatctggaagatcgtgagcaagaagaacgaactgggtgaatgtgtgcgtttcaatgttctggatccgagcagctataa
tgaatttaatgatctgctggacaagtacctggccgatgcaaaaattgcaaaaggttatgaccgtagcaaagcctggaaaaaatatttcaa
gctgaaagagaagtacgccaaggtgcgttacaacttcagcagcaccatccacaagctgcagggtagcacctatcagaatatgtattttg
acatgcgcggcctggactatttttatcgcatgaatcgtgataacgtgctgcgcctggtttatgtgggtattacccgtgcaagcgatcaggtt
 tttattctgcaggattaa
Amino acid sequence of BCH105-1 (SEQ ID NO: 6) 
MKHDLSSATEEQRYIFTDFIKQAQLALKGDSDYEVFAIQGFAGSGKTWLSA
LIIDELLELGMKVAVTSPTHKAVRVSLNMLKNNGIDTNSPLMYPGTIHHFLNLKLDHG
FADDGTADNVTTKAKLVVNKFNEALEYVDVLIVDEASMVSGELYDHALKTLGDRAK
IILFIGDSYQLLPVDDEDSSIFLKDDIFHYKLTKVVRQAEDNIIIAKSQELIKAMDQKTY
YPSVNDYFVNITEDTEGIKLLKSNVELFELYFSDFKDKMTGAYTNKVVNQFNEYIRYT
LYQETKFIADKDELVFQETYTDSKGNIIVSNGEIIEVATAKLTTDIDKFKIWKIVSKKNEL
GEAVRFNVLDPSSYNEFNDLLDKYLADAKIAKGYDRSKAWKKYFKLKEKYAKVRYN
FSSTIHKLQGSTYQNMYFDMRGLDYFYRMNRDNVLRLVYVGITRASDQVFILQD
Amino acid sequence of BCH105-2 (SEQ ID NO: 7) 
MKHDLSSATEEQRYIFTDFIKQAQLALKGDSDYEVFAIQGFAGSGKTWLSA
LIIDELLELGMKVAVTSPTHKAVRVSLNMLKNNGIDTNSPLMYPGTIHHFLNLKLDHG
FADDGTACNVTTKAKLVVNKFNEALEYVDVLIVDEASMVSGELYDHALKTLGDRAK
IILFIGDSYQLLPVDDEDSSIFLKDDIFHYKLTKVVRQAEDNIIIAKSQELIKAMDQKTY
YPSVNDYFVNITEDTEGIKLLKSNVELFELYFSDFKDKMTGAYTNKVVNQFNEYIRYT
LYQETKFIADKDELVFQETYTDSKGNIIVSNGEIIEVATAKLTTDIDKFKIWKIVSKKNEL
GEAVRFNVLDPSSYNEFNDLLDKYCADAKIAKGYDRSKAWKKYFKLKEKYAKVRYN
FSSTIHKLQGSTYQNMYFDMRGLDYFYRMNRDNVLRLVYVGITRASDQVFILQD
DNA sequence corresponding to BCH105-1 protein (SEQ ID NO: 8)

ATGAAACATGATCTGAGCAGCGCGACCGAAGAACAGCGCTATATTTTTA
CCGATTTTATCAAGCAGGCGCAGCTGGCGCTGAAAGGCGATAGCGATTATGAAGTG
TTTGCGATTCAGGGCTTTGCGGGCAGCGGCAAAACCTGGTTAAGCGCGTTAATTAT
TGATGAGCTGCTGGAACTGGGCATGAAAGTGGCCGTGACCAGCCCGACCCATAAA
GCGGTTCGTGTGAGCCTGAACATGCTGAAAAACAACGGCATTGATACCAACAGCC
CGCTGATGTATCCGGGCACCATTCATCATTTTCTGAACCTGAAACTGGATCATGGCT
TTGCGGATGATGGCACCGCGGATAACGTTACCACCAAAGCGAAACTGGTGGTGAA
CAAATTTAACGAAGCGCTGGAATATGTGGATGTGCTGATTGTGGATGAAGCGAGCA
TGGTGAGCGGCGAACTGTATGATCATGCGCTGAAAACCCTGGGCGATCGCGCGAA
AATTATTCTGTTTATTGGCGATAGCTATCAGCTGCTGCCGGTGGATGATGAAGATAG

CAGCATTTTTCTGAAAGACGACATCTTCCATTACAAGCTGACCAAGGTGGTGCGCC
AGGCGGAAGATAACATTATTATTGCGAAAAGCCAGGAGCTGATTAAAGCGATGGAT
CAGAAAACCTATTATCCGAGCGTGAACGATTATTTTGTGAACATTACCGAAGACAC
CGAAGGCATTAAACTGCTGAAAAGCAACGTGGAACTGTTTGAACTGTATTTTAGC
GATTTTAAGGACAAGATGACCGGCGCGTATACCAACAAAGTGGTGAACCAGTTTA
ACGAATATATCCGCTATACCCTGTATCAGGAAACCAAATTTATTGCGGATAAAGACG
AACTGGTGTTTCAGGAAACCTATACCGATAGCAAAGGCAACATTATTGTGAGCAAC
GGCGAAATTATTGAGGTGGCGACCGCGAAACTGACCACCGATATTGATAAATTTAA
AATCTGGAAGATCGTGAGCAAGAAGAACGAACTGGGCGAAGCGGTGCGCTTTAA
CGTGCTGGATCCGAGCAGCTATAACGAATTTAACGATCTGCTGGATAAATACCTGG
CGGATGCGAAAATTGCGAAAGGCTATGATCGCAGCAAAGCGTGGAAGAAATATTT
TAAACTGAAGGAGAAGTACGCGAAAGTGCGCTATAACTTTAGCAGCACCATTCATA
AACTGCAGGGCAGCACCTATCAGAACATGTATTTTGATATGCGCGGCCTGGATTATT
TTTATCGCATGAACCGCGATAACGTGCTGCGCCTGGTGTATGTGGGCATTACCCGCG
CGAGCGATCAGGTGTTTATTCTGCAGGATTAA

Although the specific embodiments of the present disclosure are described above, it should be understood by those skilled in the art that these are merely illustrative examples, and various changes or modifications may be made to these embodiments without departing from the principles and essence of the present disclosure. Therefore, the protection scope of the present disclosure is defined by the appended claims.

## Claims

1. A helicase mutant, wherein the helicase mutant has at least one cysteine on a surface of a three-dimensional structure of an amino acid sequence as set forth in SEQ ID NO: 1, the at least one cysteine being substituted with alanine.

2. The helicase mutant according to claim 1, wherein:
the cysteine is C133, C164, C292, C323, and/or C347; and
preferably, the amino acid sequence of the helicase mutant is as set forth in SEQ ID NO: 6.

3. The helicase mutant according to claim 2, wherein:
the helicase mutant further has at least one amino acid mutation in a pin domain, a tower domain, fragment V66-N84, and/or fragment K294-A321, the at least one amino acid mutation being substitution of an original amino acid with cysteine or an unnatural amino acid and/or cross-linking an original amino acid with at least one chemical cross-linking agent;
preferably, a mutation site in the pin domain is at least one of 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, and 134; a mutation site in the tower domain is at least one of 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, and 393; a mutation site in the fragment V66-N84 is at least one of 66, 67, 68, 69, 70, 71, 72, 73, 74, 75, 76, 77, 78, 79, 80, 81, 82, 83, and 84; and/or a mutation site in the fragment K294-A321 is at least one of 294, 295, 296, 297, 298, 299, 300, 301, 302, 303, 304, 305, 306, 307, 308, 309, 310, 311, 312, 313, 314, 315, 316, 317, 318, 319, 320, and 321; and
more preferably, the mutation site in the pin domain is at least one of G96, T97, I98, H99, H100, F101, L102, N103, L104, K105, L106, D107, H108, G109, F110, A111, D112, D113, G114, T115, A116, D117, N118, V119, T120, T121, K122, A123, K124, L125, V126, V127, N128, K129, F130, N131, E132, A133, and L134; the mutation site in the tower domain is at least one of P355, S356, S357, Y358, N359, E360, F361, N362, D363, L364, L365, D366, K367, Y368, L369, A370, D371, A372, K373, I374, A375, K376, G377, Y378, D379, R380, S381, K382, A383, W384, K385, K386, Y387, F388, K389, L390, K391, E392, and K39; the mutation site in the fragment V66-N84 is at least one of V66, T67, S68, P69, T70, H71, K72, A73, V74, R75, V76, S78, L79, N80, M81, L82, K83, and N84; and/or the mutation site in the fragment K294-A321 is at least one of K294, D295, E296, L297, V298, F299, Q300, E301, T302, Y303, T304, D305, S306, K307, G308, N309, I310, I311, V312, S313, N314, G315, E316, I317, I318, E319, V320, and A321.

4. The helicase mutant according to claim 3, wherein:
the unnatural amino acid is selected from the group consisting of 4-azido-L-phenylalanine (PAZF), 4-azido-L-phenylalanine hydrochloride (PAZF-Hcl), 4-acetyl-L-phenylalanine, 3-acetyl-L-phenylalanine, 4-acetoacetyl-L-phenylalanine, O-allyl-L-tyrosine, 3-(phenylselanyl)-L-alanine, O-2-propyn-1-yl-L-tyrosine, 4-(dihydroxyboranyl)-L-phenylalanine, 4-[(ethylthio)carbonyl]-L-phenylalanine, (2S)-2-amino-3-{4-[(propan-2-ylthio)carbonyl]phenyl}propanoic acid, (2S)-2-amino-3-{4-[(2-amino-3-thiopropionyl)amino]phenyl}propanoic acid, O-methyl-L-tyrosine, 4-amino-L-phenylalanine, 4-cyano-L-phenylalanine, 3-cyano-L-phenylalanine, 4-fluoro-L-phenylalanine, 4-iodo-L-phenylalanine, 4-bromo-L-phenylalanine, O-(trifluoromethyl)tyrosine, 4-nitro-L-phenylalanine, 3-hydroxy-L-tyrosine, 3-amino-L-tyrosine, 3-iodo-L-tyrosine, 4-isopropyl-L-phenylalanine, 3-(2-naphthyl)-L-alanine, 4-phenyl-L-phenylalanine, (2S)-2-amino-3-(naphthyl-2-ylamino)propionic acid, 6-(methylsulfanyl)norleucine, 6-oxy-L-lysine, D-tyrosine, (2R)-2-hydroxy-3-(4-hydroxyphenyl)propionic acid, (2R)-2-aminooctanoate 3-(2,2'-bipyridin-5-yl)-D-alanine, 2-amino-3-(8-hydroxy-3-quinolyl)propionic acid, 4-benzoyl-L-phenylalanine, S-(2-nitrobenzyl)cysteine, (2R)-2-amino-3-[(2-nitrobenzyl)thio]propionic acid, (2S)-2-amino-3-[(2-nitrobenzyl)oxy]propionic acid, O-(4,5-dimethoxy-2-nitrobenzyl)-L-serine, (2S)-2-amino-6-({[(2-nitrobenzyl)oxy]carbonyl}amino)hexanoic acid, O-(2-nitrobenzyl)-L-tyrosine, and 2-nitrophenylalanine; and/or
the chemical cross-linking agent is selected from the group consisting of maleimide, active ester, succinimide, azide, alkyne, phosgene-type reagent, sulfonyl chloride reagent, isothiocyanate, acyl halide, hydrazine, disulfide, vinyl sulfone, aziridine, and photosensitizer.

5. The helicase mutant according to claim 2, wherein one or more amino acid residues at the N-terminus of the amino acid sequence of the helicase mutant as set forth in SEQ ID NO: 6 are truncated; for example, amino acids M1 to A7 are truncated.

6. The helicase mutant according to claim 2, wherein the helicase mutant has at least one amino acid mutation at K2, D4, L5, K82, N83, N84, G85, I86, and D87 of the amino acid sequence as set forth in SEQ ID NO: 6, the at least one amino acid mutation being substitution of an original amino acid with a different amino acid, and preferably the different 1 amino acid being alanine.

7. The helicase mutant according to claim 2, wherein:
the helicase mutant has at least one mutation in a region interacting with a porin interface, the at least one mutation comprising substitution of an original amino acid with alanine, serine, glutamine, leucine, threonine, histidine, or glycine;
preferably, the at least one mutation in the region interacting with the porin interface is at at least one of positions 1, 2, 3, 4, 5, 12, 14, 16, 18, 21, 28, 63, 82, 124, 129, 158, 165, 189, 194, 196, 199, 202, 203, 205, 206, 207, 212, 214, 215, 218, 223, 244, 247, 261, 263, 271, 281, 289, 294, 307, 324, 331, 333, 336, 340, 341, 349, 367, 376, 382, 385, 386, 389, and 393; and
more preferably, the at least one mutation in the region interacting with the porin interface is at at least one of positions M1, K2, H3, D4, L5, Q12, Y14, F16, D18, K21, K28, K63, K82, K124, K129, K158, K165, K189, H194, K196, K199, R202, Q203, E205, D206, N207, K212, Q214, E215, K218, K223, K244, K247, K261, K263, K271, R281, K289, K294, K307, K324, K331, K333, K336, K340, K341, R349, K367, K376, K382, K385, K386, K389, and K393.

8. The helicase mutant according to claim 2, wherein:
the helicase mutant has at least one mutation in an ATP hydrolysis region, the at least one mutation comprising substitution of an original amino acid with a different charged amino acid or an amino acid with a relatively small side chain, and preferably the different charged amino acid or the amino acid with a relatively small side chain being alanine, aspartic acid, glutamine, or glycine;
preferably, the at least one mutation in the ATP hydrolysis region is at at least one of positions 40, 41, 42, 43, 44, 45, 46, 143, 144, 145, 146, 147, 148, 149, 171, 172, 173, 174, 175, 176, 177, 178, 179, 180, 181, 182, 183, 184, 185, 186, 197, 198, 199, 200, 201, 202, 203, 204, 205, 206, 207, 208, 209, 210, 211, 212, 213, 214, 215, 216, 217, 218, 219, 220, 221, 222, 223, 405, 406, 407, 408, 409, 410, 411, 412, 413, 414, 430, 431, 432, 433, 434, 435, 436, 437, 438, 439, 440, 441, 442, 443, 444, and 445; and
more preferably, the at least one mutation in the ATP hydrolysis region is at at least one of positions G40, F41, A42, G43, S44, G45, K46, E144, A145, S146, M147, V148, S149, G171, D172, S173, Y174, Q175, L176, L177, P178, V179, D180, D181, E182, D183, S184, S185, I186, L197, T198, K199, V200, V201, R202, Q203, A204, E205, D206, N207, I208, I209, I210, A211, S213, Q214, E215, L216, I217, K218, A219, M220, D221, Q222, K223, I405, H406, K407, L408, Q409, G410, S411, T412, Y413, Q414, N430, R431, D432, N433, V434, L435, R436, L437, V438, Y439, V440, G441, I442, T443, R444, and A445.

9. The helicase mutant according to any one of claims 2 to 8, wherein:
the helicase mutant has a double substitution on the amino acid sequence set forth in SEQ ID NO: 6, the double substitution being selected from the group consisting of:
L369C and D117C,
L369C and V119C,
L369C and D113C,
K373C and G114C,
K373C and D112C,
W384C and F110C,
W384C and D112C,
W384C and G114C,
L369C and T115C,
L369C and N118C,
K373C and T115C,
K373C and A116C,
K373C and D117C,
K373C and N118C,
K373C and V119C,
H108C and F388C,
V119C and W384C,
R380C and A116C,
K373C and T120C,
L369C and T121C,
A116C and 381C,
T121C and K373C,
T120C and L369C, and
T121C and 388C;
preferably, in the case of the double substitution being L369C and V119C, the helicase mutant further has a mutation selected from:
(1) single substitution selected from the group consisting of K21S, K28S, K189S, H194S, K196S, K199S, Q203S, N207S, K212S, Q214S, K218S, K223S, R202K, E215A, K2A, H3A, D4A, L5A, Y14A, F16A, D18A, K63A, K82A, K245A, K247A, K261A, R281A, K307A, K324A, K331A, K333A, K336A, K341A, K349A, K124A, K129A, K158A, K271A, K376A, K382A, K385A, K393A, and E205D; or
(2) double substitution of K72C and G315C or double substitution of E205A and D206A; or
(3) truncation of 1-st to 7-th amino acids; or
(4) truncation of M1G and an H before the amino acid sequence.

10. An isolated nucleic acid, encoding the helicase mutant according to any one of claims 2 to 9;
preferably, the nucleotide sequence of the helicase mutant is as set forth in SEQ ID NO: 8.

11. A recombinant expression vector, comprising:
a promoter; and
the nucleic acid according to claim 10, wherein:
preferably, the promoter is T7, and/or a backbone plasmid of the recombinant expression vector is PET.28a(+), PET.21a(+), or PET.32a(+).

12. A transformant, comprising a host cell and the nucleic acid according to claim 10;
preferably, the host cell is *Escherichia coli,* and more preferably BL21 (DE3), BL21 Star (DE3) pLyss, Rossata (DE3), or Lemo21 (DE3).

13. A method for preparing the helicase mutant according to any one of claims 2 to 9, the method comprising:
culturing the transformant according to claim 12 in a culture medium to ferment and produce the helicase mutant.

14. A helicase mutant-sequencing adapter complex, comprising:
the helicase mutant according to any one of claims 2 to 9; and
a sequencing adapter.

15. A kit, comprising:
the helicase mutant according to any one of claims 2 to 9 and/or the helicase mutant-sequencing adapter complex according to claim 14; and
preferably, a single-stranded DNA with an anchored molecule at a 5' end, a nanopore, a nanopore protein, an electrical signal detector, a membrane, and/or a buffer, wherein:
preferably, the anchored molecule is a hydrophobic molecule, and preferably, the anchored molecule is selected from any one or more of: lipid, fatty acid, sterol, carbon nanotube, polypeptide, protein, and/or amino acid, such as cholesterol, palmitate or tocopherol;
the nanopore is a transmembrane protein pore or a solid pore, and preferably, the nanopore is selected from the group consisting of hemolysin, MspA, MspB, MspC, MspD, FraC, ClyA, PA63, CsgG, CsgD, XcpQ, SP1, phi29 connector, InvG, GspD, and a combination thereof;
the membrane is selected from the group consisting of an amphiphilic membrane, a high molecular polymer membrane, and combinations thereof; and
the buffer is selected from the group consisting of a dihydrogen phosphate-hydrogen phosphate buffer system, a carbonic acid-sodium bicarbonate buffer system, a Tris-HCl buffer system, a HEPES buffer system, a MOPS buffer system, and combinations thereof.

16. Use of the helicase mutant according to any one of claims 2 to 9, the helicase mutant-sequencing adapter complex according to claim 14, or the kit according to claim 15 in high-throughput sequencing;
preferably, the high-throughput sequencing is nanopore sequencing.

17. A DNA unwinding method, comprising:
unwinding double-stranded DNA using the helicase mutant according to any one of claims 2 to 9, the helicase mutant-sequencing adapter complex according to claim 14, or the kit according to claim 15.

18. A sequencing method, comprising:
unwinding DNA using the DNA unwinding method according to claim 17 and simultaneously sequencing.
